# EUROPÄISCHE PATENTSCHRIFT

(11) **EP 1 095 625 B1**
(45) Veröffentlichungstag und Bekanntmachung des Hinweises auf die Patenterteilung: **14.05.2008**
(21) Anmeldenummer: 00123571.2
(22) Anmeldetag: 27.10.2000
(51) Int. Cl.: A61B 17/34

(54) **Haltesystem für Hilfsinstrumente insbesondere in der minimal invasiven Chirurgie**
Instruments support structure in particular for minimally invasive surgery
Structure de support pour instruments, en particulier pour la chirurgie avec ingérence minimale

(30) Priorität: 29.10.1999 DE 19952208; 17.03.2000 DE 10013146
(43) Veröffentlichungstag der Anmeldung: 02.05.2001
(73) Patentinhaber: Tuebingen Scientific Medical GmbH, 72074 Tuebingen (DE)
(72) Erfinder: Schwarz, Knut M., Dipl.-Ing., 72072 Tübingen (DE); Schurr, Marc O., Dr., 72074 Tübigen (DE); Buesz, Gerhard F., Prof. Dr., 72074 Tübingen (DE)
(74) Vertreter: TBK-Patent

(56) Entgegenhaltungen:
- EP-A- 0 414 130
- DE-A- 19 726 141
- US-A- 2 705 949
- US-A- 5 201 742
- US-A- 5 575 798
- US-A- 5 928 219

## Beschreibung

Die vorliegende Erfindung betrifft ein Haltesystem für chirurgische Hilfsinstrumente wie sie insbesondere in der minimal invasiven Chirurgie, vorzugsweise der sogenannten Solo-Chirurgie Anwendung finden.

Zur Durchführung komplexer chirurgischer Eingriffe in endoskopischer Technik bedarf es einer Vielzahl verschiedener Instrumente und Optiken. Zu diesen Instrumenten zählen sogenannte aktive Instrumente, die der Operateur aktiv zur Durchführung operativer Maßnahmen einsetzt und passive bzw. Hilfsinstrumente, deren Aufgabe darin besteht, den Einsatz der aktiven Instrumente erst zu ermöglichen, beispielsweise visuelle Kontrollmöglichkeiten zu schaffen oder die Bewegungsfreiheit der aktiven Instrumente im Körper zu verbessern, indem sie Gewebe oder Gewebeteile auf die Seite halten oder indem sie Gewebe für die operativen Handlungen wie Nähen, Schneiden oder Präparieren fixieren.

Im klinischen Einsatz werden die Optiken und die Hilfsinstrumente in der Regel von einem Assistenten gehalten und auf Anordnung des Operateurs geführt. Um die eher statische Arbeit der Assistenten zu erleichtern oder sogar zu ersetzen sind aus dem Stand der Technik bereits Haltearme bzw. Stative bekannt geworden, welche bereits in der Praxis ihren Einsatz finden.

Sämtliche bekannten Ausgestaltungen von Haltearmen, Stativen oder Bewegungsmechanismen sind dazu konzipiert, eine Bewegung des Instruments ausschließlich um den Eindringpunkt in die Körperwand des Patienten, auch invarianter Punkt genannt, zu ermöglichen, wobei der Eindringpunkt selbst ortsfest bleibt. D.h. sämtliche Schwenk- oder Bahnbewegungen des Instruments im Bereich seines distalen Endes werden mittels der bekannten Haltearme derart zwangsgeführt, dass das Instrument am invarianten Punkt selbst in beliebiger Richtung in der Körperwandebene unbeweglich bleibt, um somit die Körperwand, z.B. die Bauchdecke nicht zu dehnen oder gar zu zerreisen.

Die zur Erreichung dieses Ziels nötigen komplexen und aufwendigen Haltearme haben jedoch sämtlich das Problem, dass aufgrund der verwendeten Materialien eine zeitaufwendige und schwierige Sterilisation erforderlich ist. Zur Verringerung des Sterilisationsaufwands müssen die Haltearme zusätzlich mit sterilen Folien abgedeckt werden, wodurch jedoch wiederum das Einrichten der Haltearme am OP-Feld erschwert wird. Abgesehen davon sind die bekannten Haltearme nicht nur teuer sondern auch relativ anfällig gegen Beschädigung im praktischen Einsatz.

Aus der US-5,201,742 ist eine Halte- und Positioniervorrichtung für chirurgische Hilfsinstrumente und Optiken bekannt, mit einem Gestänge, an dem eine Aufnahme in Form einer eine Durchgangsbohrung aufweisenden Kugel für das Hilfsinstrument oder die Optik angeordnet ist. Die Kugel ist dabei in einer am Gestänge fixierten Lagerungsvorrichtung in Form einer Hülse oder eines Rings eingesetzt, an der eine Spannschraube vorgesehen ist. Über diese Spannschraube kann eine verstellbare Einspannkraft auf die Kugel ausgeübt werden.

Demgegenüber liegt der Erfindung die Aufgabe zugrunde, das gattungsgemäße Haltesystem derart weiter zu bilden, dass eine einfache Sterilisation und eine einfach Positionierung des Systems möglich ist.

Diese Aufgabe wird durch ein Haltesystem mit den Merkmalen gemäß dem anliegenden Patentanspruch 1 gelöst.

Die Grundüberlegung der Erfindung beruht auf der Erkenntnis, dass die Körperwand z.B. die Bauchdecke eine bestimmte Eigenelastizität aufweist, die eine minimale Spannung in Körperwand-Ebenenrichtung zulässt, ohne dass die Gefahr eines Zerreißens der Körperwand besteht. Diese Erkenntnis führt nun zu dem Erfindungsgedanken, den ortsfesten Punkt für die zwangsgeführten Bewegungen des Instruments geringfügig von der Körperwand eines Patienten zu beabstanden, wobei der Durchdringungspunkt der Körperwand bei beispielsweise einer Bahnbewegung des Instruments um den ortsfesten Punkt aufgrund vorliegender Hebelarmverhältnisse nur minimal bewegt wird. Der hierbei auftretende Bewegungsweg des Durchdringungspunkts bleibt innerhalb der Bewegungstoleranz, in der eine Schubspannung weit unterhalb der Zerreißspannung der Körperwand erzeugt wird und daher vollständig risikolos für den Patienten ist.

Das erfindungsgemäße Verlagern des ortsfesten Schwenkpunks des Instruments für dessen zwangsgeführte Bewegungen weg vom Körper des Patienten ermöglicht nunmehr auch eine erhebliche Vereinfachung des Haltearms selbst, dergestalt, dass die Mechanik nunmehr nicht wie im Stand der Technik einen im Raum befindlichen, imaginären Schwenkpunkt erzeugen muss, der dem invariablen Punkt in der Körperwand entspricht, sondern den ortsfesten Schwenkpunkt im oder nahe dem Befestigungspunkt des Instruments am Haltearm haben kann.

Diese Erfindung macht den Einsatz mehrerer Halte- und Positioniereinrichtungen möglich, wofür die Anordnung des erfindungsgemäßen Stativs besonders geeignet ist. Dieses Stativ gewährleistet eine nur geringe Beschränkung des Zugangsbereichs für den Operateur an dem OP-Tisch und schafft somit die Voraussetzung für die sogenannte Solo-Chirurgie. Dabei können natürlich nicht nur erfindungsgemäße Halte- und Positioniervorrichtungen an das Stativ angelenkt sein, sondern das erfindungsgemäße Stativ kann für sich eingesetzt und mit herkömmlichen Halte- und Positioniervorrichtungen bestückt werden.

Insbesondere im Fall des erfindungsgemäßen Stativeinsatzes aber auch bei einer herkömmlichen Anlenkung von Halte- und Positioniervorrichtungen bekannter oder erfindungsgemäßer Bauart am OP-Tisch bringt die erfindungsgemäße Halte- und/oder Arretiervorrichtung den Vorteil, dass insbesondere schwere Hilfsinstrumente nicht mehr von Assistenten gehalten werden müssen, wodurch noch mehr Bewegungsfreiraum am OP-Tische für den Operateur erhalten bleibt.

Weitere vorteilhafte Ausgestaltungen der Erfindung sind Gegenstand der übrigen Unteransprüche.

Die Erfindung wird nachstehend anhand bevorzugter Ausführungsbeispiele unter Bezugnahme auf die begleitenden Zeichnungen näher erläutert.

Es zeigen:
Fig. 1 eine Längsansicht eines Haltearms gemäß einem bevorzugten Ausführungsbeispiel der Erfindung
Fig. 2 eine Perspektivenansicht des erfindungsgemäßen Haltearms, die bei teilweise geschnittenen Bauteilen auch den Bewegungsraum eines zu haltenden Trokars darstellt,
Fig. 3 den Längsschnitt einer Klemm-/oder Arretiervorrichtung gemäß einem bevorzugten Ausführungsbeispiel der Erfindung,
Fig. 4 die Seitenansicht eines OP-Felds mit einem als Anlenkschiene ausgebildeten Stativ gemäß einem bevorzugten Ausführungsbeispiel der Erfindung und
Fig. 5 die Draufsicht auf das OP-Feld gemäß Fig. 4.

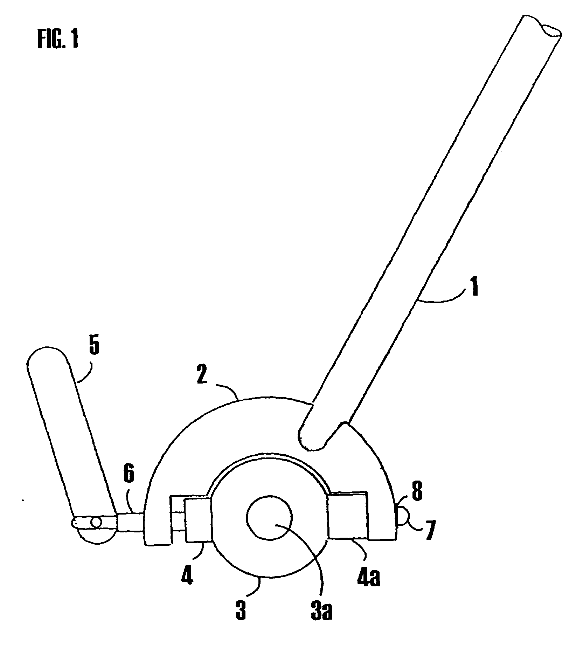

Gemäß der Figur 1 besteht der erfindungsgemäße Haltearm bzw. die Halte- und Positioniervorrichtung für chirurgische Hilfsinstrumente aus einem Haltestab 1 aus einem Metallmaterial in Hohlprofil oder einem Kunststoffmaterial, vorzugsweise jeweils mit bestimmten elastischen Eigenschaften, an dessen einem Endabschnitt ein Halterrahmen 2 fest fixiert ist. Der Halterrahmen 2 ist gemäß der Figur 1 zu einem U-förmigen Joch geformt, wobei an den Endabschnitten der hierdurch entstehenden im wesentlichen parallel beabstandeten Schenkel Anlenksockel 2a, 2b zur Anordnung einer Lagerungseinrichtung ausgebildet sind.

Die Anlenksockel 2a, 2b sind vorliegend rechtwinklige Ausnehmungen oder Aussparrungen, wodurch plane Oberflächen entstehen, die einen vorbestimmten Parallelabstand zueinander aufweisen. Des weiteren sind die Anlenksockel 2a, 2b von jeweils einer Bohrung bzw. einem Durchgangsloch durchdrungen, die im wesentlichen im Mittelpunkt der jeweiligen Anlenksockel 2a, 2b vorgesehen sowie koaxial zueinander ausgerichtet sind.

In der gemäß Figur 1 linksseitigen Bohrung ist ein Lagerungs- bzw. Betätigungszapfen 6 eines ersten Haltestempels 4 eingesetzt. Der Betätigungs- und Lagerungszapfen 6 ragt dabei über die außenseitige Oberfläche des Halterrahmens 2 vor und bildet an seinem außenseitigen Endabschnitt eine Anlenkstelle für einen Betätigungshebel 5. Der erste Haltestempel 4 in Form eines Zylinderkolbens hat an seiner dem Lagerungszapfen 6 abgewandten Stirnseite eine konkave Eingriffsfläche.

An dem gegenüberliegenden, gemäß der Figur 1 rechtsseitigen Anlenksockel 2a ist ebenfalls ein zweiter Haltestempel 4a angeordnet. Dieser zweite Haltestempel 4a besteht aus einem zylinderförmigen Kolben, der an seiner einen Stirnseite eine konkave Eingriffsfläche ausbildet und an seiner gegenüberliegenden Stirnseite einen Lagerungszapfen 7 hat, welcher in die Bohrung des rechtsseitigen Anlenksockels 4a eingeführt ist und auf der Außenseite des Rahmens 2 vorsteht. In diesem vorstehenden Abschnitt ist eine Ringnut (nicht weiter gezeigt) ausgebildet, in die ein Haltering 8 eingesetzt ist. (Alternativ kann der Lagerungszapfen 7 auch einfach in die Bohrung eingeklebt, gepresst oder geschraubt sein).

Zwischen den beiden Haltestempeln 4, 4a befindet sich eine eine Aufnahme für das Instrument bildende Aufnahmekugel 3, die eine durchgehende Zentralbohrung 3a aufweist. Die Kugel 3 hat dabei einen Radius, der kleiner als der Innenradius des U-förmigen Halterrahmens 2 ist, so dass sie ausschließlich von den beiden sich gegenüberliegenden Haltestempeln 4, 4a in Position gehalten wird.

Wie ferner aus der Figur 1 zu entnehmen ist, lässt sich der gemäß Figur 1 linksseitige Haltestempel 4 über den Betätigungshebel 5 entlang des Lagerungszapfens 6 axial verschieben, um hierdurch die Einspannkraft, welche auf die Kugel 3 aufgebracht wird, zu variieren und damit die Klemmkraft soweit zu erhöhen, dass die hierbei entstehende Reibungskraft zwischen der Kugeloberfläche und den konkaven Flächen der Haltestempel 4, 4a eine Bewegungsarretierung der Kugel 3 bewirkt. An dieser Stelle sei darauf hingewiesen, dass der eine, mit dem Betätigungshebel 5 gekoppelte Lagerungszapfen 4 beispielsweise als Schraube ausgebildet sein kann, welche über den Betätigungshebel 5 ein- und ausgedreht wird. Alternativ kann dieser Lagerungszapfen 4 als Bolzen mit einer diesen umgebenden Feder ausgebildet sein, welche den Lagerungszapfen und den einstückig daran ausgebildeten Haltestempel 4 in Richtung Kugel 3 vorspannt, wobei der Betätigungshebel 5 beispielsweise auf die Feder zur Einstellung der Vorspannkraft einwirkt.

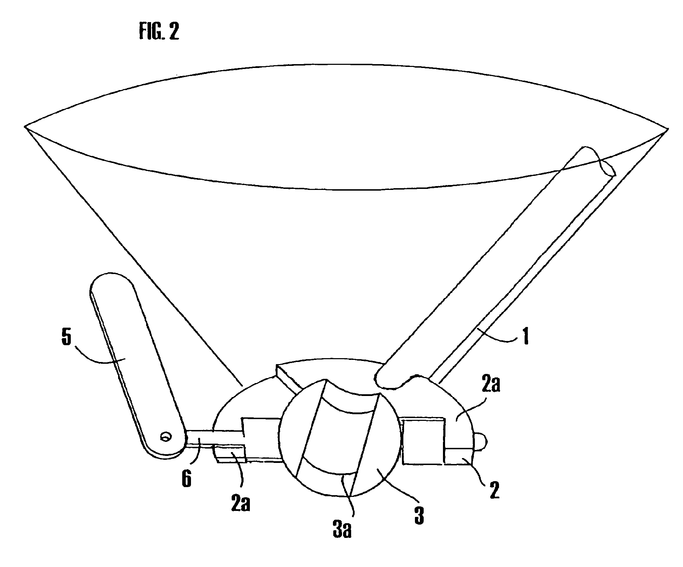

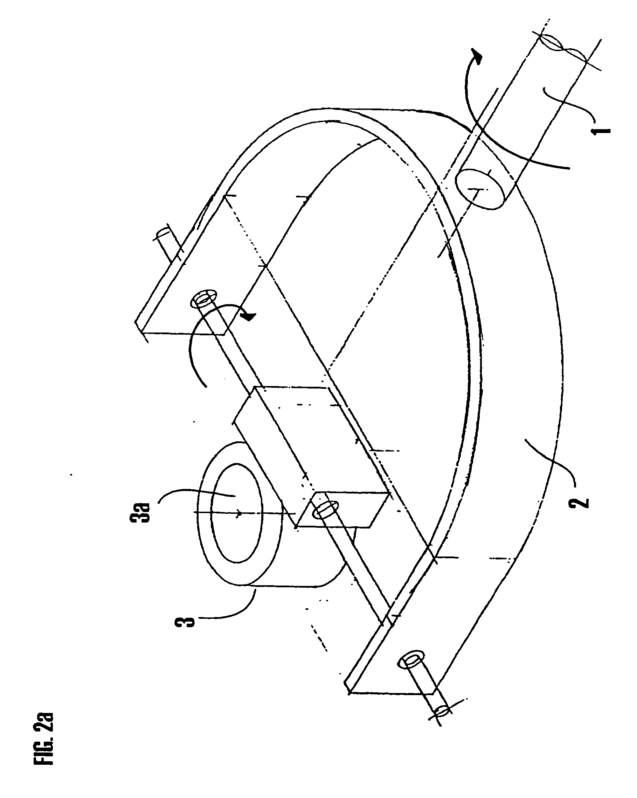

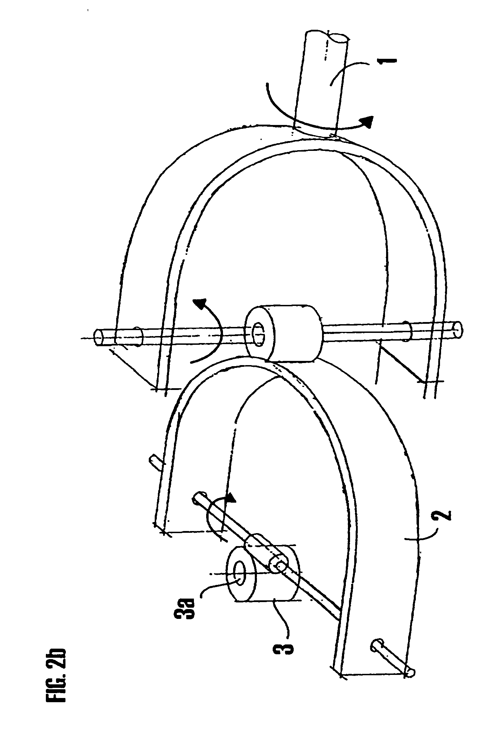

In Figur 2 ist der Haltearm 1 gemäß dem bevorzugten Ausführungsbeispiel der Erfindung in teilweise aufgebrochener Darstellung schräg von vorn abgebildet.

Wie hieraus zu entnehmen ist, kann die Kugel 3 innerhalb eines vorbestimmten Schwenkbereichs zwischen den beiden Haltestempeln 4, 4a gedreht bzw. geschwenkt werden, wobei ein in die zentrale Durchgangsbohrung 3a eingestecktes Instrument (nicht gezeigt) eine Bewegung ausführen kann, die durch einen in Figur 2 schematisch dargestellten Kegel beschrieben ist. Der Schwenkpunkt der Kugel liegt dabei auf der durch die beiden Haltestempel 4, 4a definierten Mittelachse zwischen den beiden Haltestempeln 4, 4a.

Wie ferner in der Fig. 2 zu sehen ist, sind am Innenumfang der zentralen Durchgangsbohrung 3a zwei axial beabstandete Radialnuten ausgebildet, in die Dichtungsringe eingesetzt sind. Diese Dichtungsringe üben auf das in die zentrale Durchgangsbohrung eingesetzte Instrument, beispielsweise ein Trokar, eine Klemmkraft durch die Elastizität des Dichtungsringmaterials (z.B. Gummi) aus, um das Instrument in der Kugel zu fixieren. Zusätzlich können Fixiereinrichtungen, z.B. eine Klemmschraube, vorgesehen sein, die in die Kugel eingedreht ist und auf das Instrument einwirkt.

Zur Funktionsweise des erfindungsgemäßen Haltearms lässt sich folgendes ausführen:

Zuerst wird das zu verwendende Hilfsinstrument in Form beispielsweise eines Trokars in die in der Kugel 3 ausgebildete Durchgangsbohrung 3a eingesteckt. Anschließend wird der Trokar in den Körper eines zu behandelnden Patienten beispielsweise durch dessen Bauchdecke als Körperwand eingeführt, indem die Kugel 3 oberhalb des Eindringpunkts auf die Bauchdecke aufgesetzt und der Trokar axial verschoben wird sowie in einer für den Operateur günstigen Lage positioniert wird. Hierbei gleitet und rotiert der Trokar unter Überwindung der über die Dichtungsringe auf ihn ausgeübten Klemmkraft innerhalb der Durchgangsbohrung, während die auf der Bauchdecke des Patienten aufliegenden Kugel 3 zwischen den beiden Haltestempeln 4, 4a dreht und schwenkt. Sobald das Hilfsinstrument die gewünschte Lage eingenommen hat, wird es durch die Dichtungsringe gehalten, wobei natürlich auch die zusätzliche Fixier- bzw. Arretierungseinrichtung, beispielsweise die Klemmschraube in der Kugel vorgesehen sein kann, die folglich seitlich in die Durchgangsbohrung mündet. Zur abschließenden Fixierung des Hilfsinstruments in der bestimmten Position betätigt der Operateur nunmehr den Betätigungshebel 5, um hierdurch, die über die beiden Haltestempel 4, 4a auf die Kugel 3 aufgebrachte Haltekraft zu Erhöhen und dadurch die Kugel 3 zwischen den Haltestempeln 4, 4a einzuklemmen.

Will der Operateur die Position des Hilfsinstruments verändern, muss lediglich die auf die Kugel 3 aufgebrachte Halte- bzw. Klemmkraft durch entsprechendes Betätigen des Betätigungshebels 5 geringfügig reduziert werden, soweit, dass eine gehemmte Bewegung der Kugel 3 zwischen den Haltestempeln 4, 4a ermöglicht wird. Hierauf kann das Hilfsinstrument gedreht und geschwenkt werden, wobei es eine in der Figur 2 dargestellte kegelförmige Bewegung ausführt.

Der Schwenkpunkt des Hilfsinstruments liegt dabei im wesentlichen auf der die beiden Haltestempel 4, 4a miteinander verbindenden Mittelachse in etwa in der Mitte des Abstandes zwischen den beiden Haltestempeln 4, 4a.

Wie vorstehend bereits ausgeführt wurde, wird bei Einführen des Hilfsinstruments in den Körper des Patienten beispielsweise durch dessen Bauchdecke die Kugel 3 an die Oberfläche der Bauchdecke des Patienten nahe herangeführt und gegebenenfalls sogar auf dieser abgestützt. Der Abstand zwischen dem vorstehend beschriebenen Schwenkpunkt des Hilfsinstruments, welcher nur im vorliegenden Ausführungsbeispiel jedoch nicht notwendigerweise dem Schwenkmittelpunkt der Kugel 3 entspricht, zu der Bauchdeckenoberfläche beträgt dabei je nach Durchmesser der Kugel 3 zwischen 1 bis 3 Zentimetern. Aufgrund dieses geringen Abstandes wird bei einer entsprechenden Lageveränderung des bereits eingeführten Hilfsinstruments jener Punkt, in welchem das Hilfsinstrument die Bauchdecke des Patienten durchstößt, nur relativ gering in Bauchdeckenebene verschoben, wobei eine nur geringe Schubspannung in der Bauchdecke entsteht.

Zusätzlich kann der Stab 1 wie vorstehend ausgeführt eine gewisse Eigenelastizität aufweisen, wodurch bei einer entsprechenden schwenkenden Bewegung des bereits eingeführten Hilfsinstruments und der hierbei auf die Bauchdecke aufgebrachten Schubspannung eine den Stab 1 biegende Reaktionskraft auftritt, die den Stab 1 elastisch verformt. D.h., dass in diesem Fall der Schwenkpunkt des Hilfsinstruments, d.h. der Schwenkpunkt der Kugel 3 bezüglich des Halterrahmens 2 zwar ortsfest, bezüglich des Eindringpunkts in den Körper des Patienten jedoch verschieblich ist und zwar im Rahmen der elastischen Deformation des Stabs 1, wodurch die auf die Bauchdecke aufgebrachte Schubspannung in Folge des sich zusätzlichen, Verschiebens des Schwenkpunkts bezüglich des Durchdringungspunkts der Bauchdecke teilweise kompensiert und somit weiter verringert wird.

Abschließend sei darauf hingewiesen, dass nicht notwendigerweise eine beidseits eingespannte Kugel zur Aufnahme von Hilfsinstrumenten verwendet werden muss. ermöglicht. Wesentlich ist nur, dass sich der Schwenkpunkt der Kugel in zumindest eine Bewegungsrichtung im wesentlichen zwischen zwei Anlenkpunkten am entsprechend geformten Halterahmen des Haltearms befindet, wodurch sich die Gesamtkonstruktion gegenüber dem Stand der Technik wesentlich vereinfacht. Die Kugel muss demzufolge nicht unbedingt zwischen zwei Stempeln eingespannt sein, sondern kann auch in einer entsprechend geformten Lagerschale sitzen.

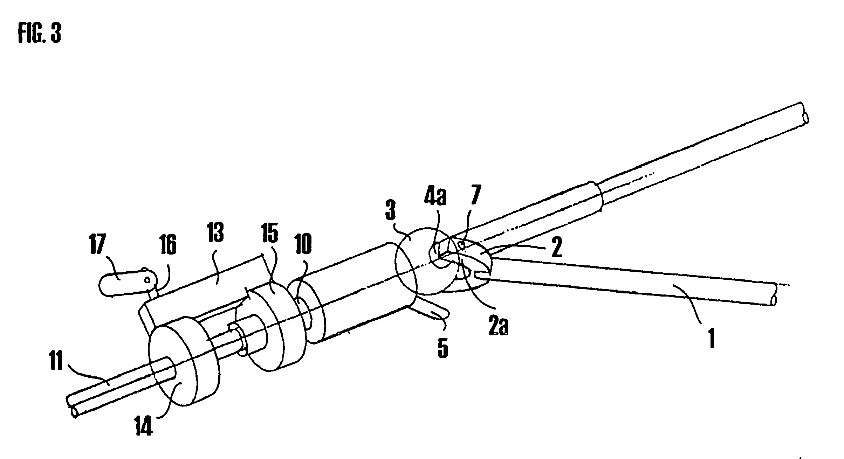

Gemäß der Figur 3 wird eine Halte- und/oder Arretiervorrichtung zur Fixierung eines Hilfsinstruments vorstehend genannter Bauart in einem Trokar dargestellt, welche insbesondere in Kombination mit einer Halte- und Positioniervorrichtung, wie sie beispielsweise vorstehend anhand des in den Figuren 1 und 2 gezeigten Haltearms beschrieben ist, zur besseren Positionierbarkeit von chirurgischen Hilfsinstrumenten beiträgt.

Gemäß der Figur 3 besteht ein Trokar 10 im allgemeinen aus einem langgestreckten zylindrischen Aufnahmerohr, in welches ein chirurgisches Instrument, ein Hilfsinstrument oder eine Optik 11 einführbar ist. Bei einer minimal invasiven Operationstechnik werden demzufolge die zu verwendenden Instrumente, Hilfsinstrumente und Optiken 11 mit Hilfe von derartigen Trokaren in den Körper eines zu behandelnden Patienten eingeführt. Der Trokar hat dabei ferner die Funktion, einen Zugang zu der Operationsstelle innerhalb des Körpers bei einem Instrumentenwechsel offen zu halten, wobei er durch geeignete Mechanik das Austreten von Insulationsgasen verhindert.

Um chirurgische Maßnahmen über den Trokar durchführen zu können, müssen die Instrumente innerhalb des Trokars sowohl translatorisch als auch rotatorisch beweglich sein, wobei insbesondere bei der Verwendung von Hilfsinstrumenten diese in einer vorbestimmten Position auch arretiert werden müssen.

Zum einen werden folglich die eingesetzten Trokare mittels Halte- und Positioniervorrichtungen, wie sie beispielsweise vorstehend anhand eines bevorzugten erfindungsgemäßen Ausführungsbeispiels beschrieben wurden, in einer bestimmten Relativlage zu der Eindringstelle vorzugsweise in die Bauchdecke eines Patienten gehalten. Zum anderen besteht aber auch die Notwendigkeit, das in den Trokar eingeführte Instrument nach Erreichen einer vorbestimmten Relativlage des Instruments bezüglich des Trokars ebenfalls in dieser Relativlage zu arretieren.

Aus dem Stand der Technik ist es bekannt, Trokare an geeigneten axial beabstandeten Stellen mit Dichtungsvorrichtungen beispielsweise in Form von Dichtungslippen oder -ringen auszustatten, um, wie vorstehend angedeutet wurde, das Austreten von Gasen zu verhindern. Derartige Dichtungsvorrichtungen üben natürlich auch eine gewisse Reibungskraft auf das in den Trokar eingeführte Instrument aus. Indessen sind die aus dem Stand der Technik bekannten Dichtungsvorrichtungen nicht dazu geeignet, insbesondere schwere Instrumente, wie beispielsweise Optiken, zu halten, d.h. eine translatorische oder rotatorische Kriechbewegung derartiger Optiken innerhalb des Trokars zu verhindern.

Um daher ein langsames Eindringen von Hilfsinstrumenten oder Optiken in den Körper eines zu behandelnden Patienten zu verhindern, müssen insbesondere schwere Instrumente nach wie vor von Assistenten des Operateurs im Operationssaal gehalten werden.

Die Erfindung sieht zur Lösung dieses Problems gemäß der Figur 3 eine bügelförmige Halte- und/oder Arretiervorrichtung 12 vor, bestehend aus einem Steg 13, an dessen Endseiten zwei durch den Steg 13 im wesentlichen parallel beabstandete Schenkel 14, 15 einstückig ausgebildet sind. Der gemäß der Figur 3 rechtsseitige eine Schenkel 15 weist dabei ein Durchgangsloch auf, dessen Durchmesser im wesentlichen dem Außendurchmesser des Trokars 10 entspricht bzw. geringfügig größer ist. Der andere, gemäß der Figur 3 linksseitige Schenkel 14 ist ebenfalls mit einem Durchgangsloch versehen, dessen Durchmesser dem Außendurchmesser des Instrumentes 11 gleichkommt bzw. geringfügig größer ist. Vorzugsweise hat der eine, mit dem Durchgangsloch zur Aufnahme des Trokars 10 ausgebildete Schenkel 15 einen Scharnier- oder Klappmechanismus, der ein Öffnen des Durchgangslochs ähnlich einer Schelle ermöglicht, wodurch der Trokar 10 in das Durchgangsloch seitlich einsetzbar ist. Beispielsweise kann der Schenkel 15 zweigeteilt sein, wobei die beiden Schenkelteile in zusammengebautem Zustand das Durchgangsloch ausbilden und wobei die beiden Schenkelteile voneinander beabstandet oder getrennt werden können, um an dem Außenmantel des Trokars 10 montiert zu werden. Hierfür können die beiden Schenkelteile vorzugsweise mittels Schrauben verbunden sein.

Der andere Schenkel 14 mit dem zur Aufnahme und Führung des Instruments ausgebildeten Durchgangsloch hat zusätzlich einen Arretiermechanismus beispielsweise in Form einer in Figur 3 nur andeutungsweise dargestellten Klemmschraube 16 mit Betätigungshebel 17, die seitlich in den Schenkel 14 oder oberhalb des Schenkels 14 in den Steg 13 eingedreht ist und in das Durchgangsloch seitlich mündet.

Zur Funktionsweise der in Figur 3 dargestellten Halte- und/oder Arretiervorrichtung wird folgendes ausgeführt:

Zuerst wird die Halte- und/oder Arretiervorrichtung an dem außenseitigen Ende des in einen Patienten einzuführenden Trokars 10 fixiert, indem der Trokar 10 endseitig in das Durchgangsloch mit großem Durchmesser eingesetzt wird. Hierzu wird das Durchgangsloch durch entsprechende Betätigung des Schaniermechanismuses geöffnet, beispielsweise entsprechend einer Rohrschelle, und der Trokar 10 seitlich in das nunmehr aufgeweitete bzw. seitlich geöffnete Durchgangsloch eingesetzt. Anschließend wird der Schaniermechanismus wieder zugeklappt und der Trokar 10 in dem Durchgangsloch festgeklemmt.

Die Klemmkraft ist dabei ausreichend, dafür, ein Verrutschen der Halte- und/oder Arretiervorrichtung längs des Trokars 10 bzw. ein Verdrehen der Halte- und/oder Arretiervorrichtung um den Trokar 10 zu verhindern. Die Klemmkraft ist jedoch klein genug, um ein Einquetschen des Trokars 10 zu vermeiden, wodurch der innere Querschnitt des Trokars 10 unverändert bleibt.

Anschließend wird das chirurgische Instrument, das Hilfsinstrument oder die Optik 11 in den Trokar 10 eingeführt, indem das Instrument 11 zuerst durch das Durchgangsloch mit kleinem Durchmesser im Schenkel 14 eingesteckt wird, dessen Mittelachse mit der Mittelachse des zuvor arretierten Trokars 10 fluchtet. Der Arretiermechanismus im Bereich des Durchgangslochs mit kleinem Durchmesser, vorzugsweise die Klemmschraube 16 ist dabei geöffnet, so dass ein reibungsfreies Einführen des Instruments 11 weiter in den Trokar 10 möglich ist.

Sobald das Instrument 11 eine bezüglich des Trokars 10 bestimmte Position einnimmt, wird der Arretiermechanismus, vorliegend die Klemmschraube 16 mittels des Hebels 17 betätigt, um eine bestimmte Klemmkraft in Radialrichtung des Durchgangslochs mit kleinem Durchmesser auf das Instrument 11 auszuüben und dieses in dem Durchgangsloch translatorisch sowie rotatorisch zu arretieren.

Je nach Ausbildung des Arretiermechanismuses, kann der Oberflächenbereich, in welchem die Klemm- bzw. Arretierkraft auf das Instrument 11 aufgebracht wird, derart vergrößert werden, dass trotz Verringerung der Arretierkraft die hierbei erzeugte Reibkraft ausreicht, um ein kriechendes Verschieben des Instruments 11 innerhalb des Trokars 10 oder ein Verdrehen des Instruments 11 bezüglich des Trokars 10 auszuschließen und gleichzeitig ein Einquetschen des Instruments 11 zu vermeiden.

Es sei ferner darauf hingewiesen, dass der Arretiermechanismus nicht notwendigerweise eine Schraube sein muss. Beispielsweise könnte in das Durchgangsloch mit kleinem Durchmesser eine konisch geformte, aus dem Stand der Technik bekannte Spreizmuffe eingedreht sein, deren innerer Querschnitt sich bei deren Ein- und Ausdrehen verengt und erweitert, um somit eine variable radiale Klemmkraft auf das in die Spreizmuffe eingesteckte Instrument 11 auszuüben. Auch muss die erfindungsgemäße Halte- und/oder Arretiervorrichtung nicht notwendiger Weise als ein externes Bauteil zur nachträglichen Anbringung an bekannte Trokare ausgebildet sein. Vielmehr ist es auch möglich, die erfindungsgemäße Halte- und/oder Arretiervorrichtung als Bestandteil des Trokars, vorzugsweise einstückig oder fest mit diesem verschraubt auszubilden, also einen neue Trokarkonstruktion zu schaffen, wonach die erfindungsgemäße Halte- und/oder Arretiervorrichtung am Trokar bereits integriert ist. In diesem Fall könnte dann auf den vorstehend beschriebenen Scharnier- oder Klappmechanismus vollständig verzichtet werden.

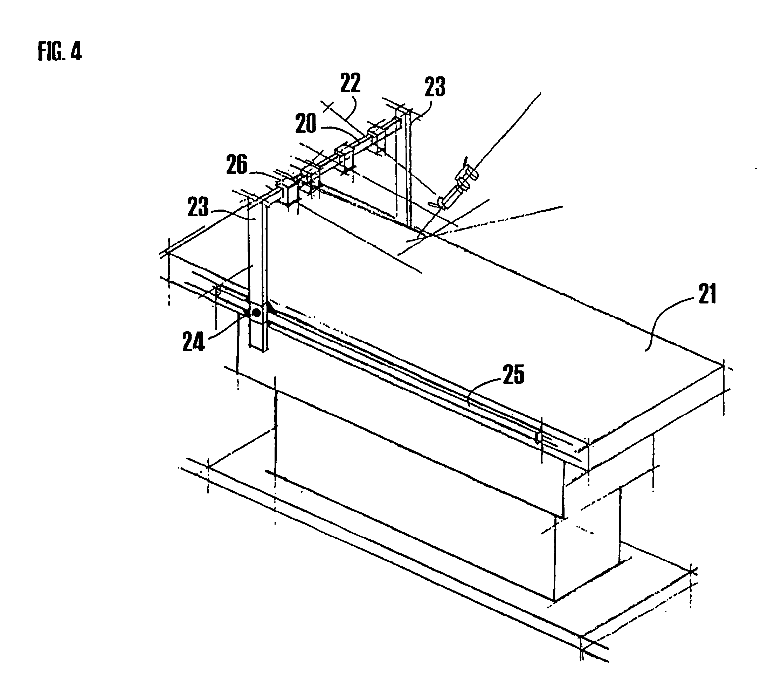

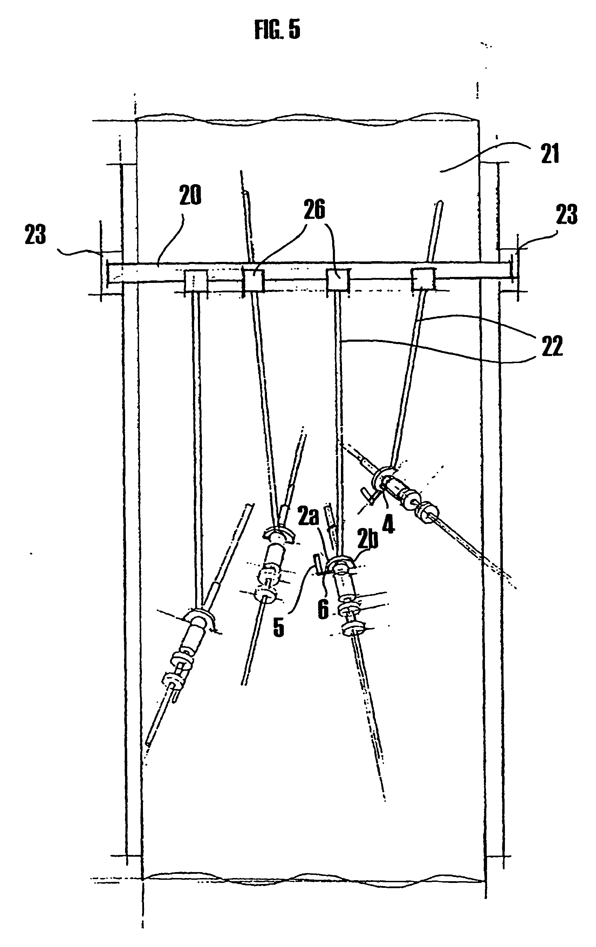

Schließlich wird gemäß der Fig. 4 und 5 ein Haltesystem bzw. Stativ zur Anlenkung von Halte- und Positioniervorrichtungen insbesondere gemäß der Fig. 1 und 2 an einem OP-Tisch dargestellt, wobei in den von den Halte- und Positioniervorrichtungen gehaltenen Trokaren chirurgische Instrumente, Hilfsinstrumente und/oder Optiken geführt sind, die optional mittels Halte- und Arretiervorrichtungen vorzugsweise gemäß der Fig. 3 in den Trokaren arretierbar sind.

Grundsätzlich ist für die Zukunft zu erwarten, dass mit gesteigertem Einsatz minimal invasiver Chirurgietechniken immer mehr entsprechend geformter Instrumente, Optiken sowie Hilfsinstrumente für jeden operativen Eingriff verwendet werden müssen, die letztlich die eingangs genannte Solo-Chirurgie ermöglichen. Derartige Hilfsinstrumente sowie Optiken müssen natürlich einen ortsfesten Halte- oder Montagepunkt haben, um eine Positionierung bezüglich eines bestimmten Punkts innerhalb des Körpers eines zu behandelnden Patienten einnehmen zu können.

Beispielsweise die vorstehend anhand der Fig. 1 und 2 beschriebene Halte- und Positioniervorrichtung muss demzufolge irgendwo im Operationssaal, vorzugsweise am OP-Tisch anmontiert werden. Dabei liegt es auf der Hand, dass der OP-Tisch selbst nur begrenzt Raum zur Befestigung von gattungsgemäßen Halte- und Positioniervorrichtungen bietet, da der Rundumzugang zur Operationsstelle am Patienten für den Operateur nicht versperrt werden darf.

Bisher wurden gattungsgemäße Halte- und Positioniervorrichtungen seitlich am OP-Tisch angeschraubt, wodurch der Freiraum für den Operateur eingeengt wird. Solange jedoch nur eine geringe Zahl von Hilfsinstrumenten mittels derartiger Halte- und Positioniervorrichtungen pro Operation verwendet werden, stellt diese Anordnungsvariante keine Probleme für den Operateur dar. Um nunmehr die Zahl der Hilfsinstrumente zu erhöhen, eignen sich die am OP-Tisch zur Verfügung stehenden Montagemöglichkeiten nicht mehr.

Zur Lösung dieses Problems schlägt die Erfindung gemäß der Fig. 4 und 5 die Anordnung eines Stativs mit einer Halte-/Führungsschiene 20 vor, die portal- oder jochartig (d.h. galgenförmig) über den OP-Tisch 21 geführt wird und welche als Anlenkschiene für Halte- und Positioniervorrichtungen 22 dient.

Die Halte-/Führungsschiene 20 besteht dabei aus einem Träger, der vorliegend durch zwei endseitig am Träger 20 angeordnete Abstützstreben 23 am OP-Tisch montiert ist. Hierdurch entsteht eine Art Portal, dessen Höhe ausreicht, über einen auf dem OP-Tisch 21 liegenden Patienten hinweggefahren zu werden. Alternativ kann der Träger 20 jedoch auch nur eine Abstützstrebe endseitig aufweisen und an seinem freien Ende frei über den OP-Tisch auskragen.

Gemäß der Fig. 5 sind die zwei Abstützstreben 23 als U-Profile ausgebildet und bilden so zwischen den Schenkeln jedes U-Profils eine Art Führungsschiene, in denen die Enden des Trägers 20 höhenverschieblich sowie arretierbar geführt sind. Auf diese Weise lässt sich die lichte Höhe des Portals variieren. An den freien Enden der Abstützstreben 23 sind gemäß der Fig. 4 Montagemittel, beispielsweise Schraub- oder Rastelemente vorgesehen, die entsprechend den am OP-Tisch vorgesehenen

Befestigungseinrichtungen ausgebildet sind. Vorliegend sind an den freien Enden der Abstützstreben 21 jeweils eine Schelle 24 angeordnet, die mit einer im allgemeinen längs des OP-Tischs beidseits verlaufenden Haltestange 25 in Klemmeingriff bringbar ist, sodass das Portal entlang der beiden Haltestangen 25 verschoben werden kann.
Wie insbesondere aus der Fig. 5 zu entnehmen ist, dient vorliegend der Träger 20 zur Halterung von insgesamt vier Halte- und Positioniervorrichtungen 22, an deren freien Enden Aufnahmen für das Anbringen von chirurgischen Hilfsinstrumenten und Optiken vorgesehen sind. Vorzugsweise entspricht die Konstruktion jedes der vier Halte- und Positioniervorrichtungen sowie der Aufnahmen jener gemäß der eingangs beschriebenen Fig. 1 und 2, wobei natürlich auch andere, bereits aus dem Stand der Technik bekannte Halte- und Positioniervorrichtungen mit der erfindungsgemäßen Halte- und Positionierschiene 20 kombinierbar sind.

Zur Montage der Halte- und Positioniervorrichtungen 22 an dem Träger 20 sind Klemmelemente 26 vorgesehen, die entsprechend einer Spannpratze den Träger 20 zumindest partiell umgreifen und sich so an diesen festklammern. Alternativ hierzu ist es auch möglich Hülsen vorzusehen, die schlittenförmig am Träger 20 geführt und mittels einer Arretierschraube feststellbar sind. Jedes Klemmelement 26 bildet ferner eine Aufnahme beispielsweise in Form einer Aufnahmebohrung, in die ein Haltestab jeder Halte- und Positioniervorrichtung 22 eingesteckt wird.

(Als besonders vorteilhaft hat es sich erwiesen, den Träger 20 entsprechend der Haltestangen 25 am OP-Tisch zu gestalten, welche in der Regel ein T-Profil hat. Bei dieser Ausgestaltung müssen keine Änderungen der Klemmelemente 26 an den Halte- und Positioniervorrichtungen 22 vorgenommen werden, die an die Haltestangen 25 konstruktiv angepasst sind).

Zusätzlich kann der Träger 20 einen Teleskopmechanismus (nicht weiter gezeigt) aufweisen, um dessen Länge variieren zu können. In diesem Fall müssen die freien Enden des Trägers 20 an den Abstützstreben 23 zusätzlich schwenkbar gelagert sein. Dies hat den Vorteil, dass der Träger 20 in einem beliebigen Winkel quer zum OP-Tisch 21 ausgerichtet werden kann, so dass an dem Träger 20 zu montierende Positionier- und Haltevorrichtungen 22 bei deren Verschiebung längs des nunmehr schräg verlaufenden Trägers 20 sowohl in Querrichtung als auch in Längsrichtung des OPTisches 21 in einem Vorgang positionierbar sind.

Abschließend sei darauf hingewiesen, dass der Bereich am OP-Tisch im wesentlichen in einen sterilen und unsterilen Raumabschnitt aufgeteilt ist, die durch entsprechende Abhängtücher voneinander separiert werden. Es hat sich dabei als vorteilhaft erwiesen, das erfindungsgemäße Portal in dem unsterilen Raumbereich zu positionieren, in dem das Portal mit entsprechenden sterilen Tüchern abgedeckt und somit von Aktionsbereich der Operateurs (steriler Raumbereich) isoliert wird.

## Patentansprüche

1. Halte- und Positioniervorrichtung für chirurgische Hilfsinstrumente und Optiken mit einem mechanischen Gestänge (1), an dessen freiem Endabschnitt eine Aufnahme in Form einer eine Durchgangsbohrung (3a) aufweisenden Kugel (3) für das Hilfsinstrument oder die Optik angeordnet ist, die in einer am mechanischen Gestänge (1) fixierten Lagerungsvorrichtung eingesetzt ist, welche eine verstellbare Einspannkraft auf die Kugel (3) ausübt, **dadurch gekennzeichnet, dass** die Lagerungsvorrichtung einen im wesentlichen U-förmigen Halterahmen (2) hat, an dessen freien Schenkeln axial fluchtende Durchgangsbohrungen zur Aufnahme von Einspannmitteln ausgebildet sind und der einen lagerschalenartigen Kugelsitz aufweist, in dem die Kugel (3) mittels der Einspannmittel eingespannt ist.

2. Halte- und Positioniervorrichtung Anspruch 1 **dadurch gekennzeichnet, dass**
die Einspannmittel aus zwei axial fluchtenden Haltestempeln (4, 4a) besteht, welche die Kugel (3) zwischen sich einspannen.

3. Halte- und Positioniervorrichtung nach Anspruch 2,
**dadurch gekennzeichnet, dass**
zumindest einer der Stempel (4) mittels einer Handhabe (5) axial verschiebbar ist, um die Einspannkraft auf die Kugel (3) zu variieren.

4. Halte- und Positioniervorrichtung nach einem der Ansprüche 2 oder 3, **dadurch gekennzeichnet, dass** zumindest einer der Stempel (4) in Axialrichtung mittels eines Vorspannmittels insbesondere einer oder mehrer Federn, eines hydraulischen, pneumatischen, elektrischen, magnetischen oder einer Kombination vorstehend genannter Mittel vorgespannt ist.

5. Halte- und Positioniervorrichtung nach einem der vorstehenden Ansprüche, **dadurch gekennzeichnet, dass** der Mittelpunkt der Kugel (3) im wesentlichen auf der Mittelachse der Durchgangsbohrung (3a) liegt.

6. Halte- und Positioniervorrichtung nach einem der vorstehenden Ansprüche, **dadurch gekennzeichnet, dass** die Aufnahme eine Hülse ist, an deren Außenmantel die Kugel (3) fixiert ist.

7. Halte- und Positioniervorrichtung nach einem der vorstehenden Ansprüche **gekennzeichnet durch** eine Arretiervorrichtung für das chirurgische Hilfsinstrument bestehend aus einer im wesentlichen U- oder bügelförmige Halterung (12) mit zwei parallel beabstandeten Schenkeln (14, 15), die erste und zweite Durchgangsöffnungen axial fluchtend ausbilden und zumindest einem Klemmmittel (16) zur individuellen, zumindest partiellen Verstellung des Durchmessers eines der ersten und zweiten Durchgangsöffnungen.

8. Halte- und Positioniervorrichtung nach Anspruch 7,
**dadurch gekennzeichnet, dass**
die erste Durchgangsöffnung einen größeren Durchmesser aufweist als die zweite Durchgangsöffnung.

9. Halte- und Positioniervorrichtung nach einem der Ansprüche 7 und 8, **dadurch gekennzeichnet, dass** das zumindest eine Klemmmittel (16) eine Klemmschraube ist, die in die zweite Durchgangsöffnung mit kleinerem Durchmesser mündet.

10. Halte- und Positioniervorrichtung nach einem der Ansprüche 7 bis 9, **dadurch gekennzeichnet, dass** die zweite Durchgangsöffnung radial aufweitbar oder schellförmig öffenbar ist, um einen seitlichen Zugang in die Durchgangsöffnung für ein Einsetzen eines Trokars zu schaffen.

11. Halte- und Positioniervorrichtung nach einem der vorstehenden Ansprüche, **gekennzeichnet durch** ein Stativ zur Anlenkung einer Anzahl von Halte- und Positioniervorrichtungen mit
einer Anlenkschiene (20), die zumindest an ihrem einen Endabschnitt eine Abstützstrebe (23) hat, an der Befestigungsmittel (24) zur portalartig einen OP-Tisch überspannenden Befestigung des Stativs vorgesehen sind.

12. Halte- und Positioniervorrichtung nach Anspruch 11, **dadurch gekennzeichnet, dass**
die Anlenkschiene (20) an der einen oder zwei endseitig an der Schiene angeordneten Abstützstreben (23) höhenverschiebbar und arretierbar ist.

13. Halte- und Positioniervorrichtung nach Anspruch 11 oder 12, **dadurch gekennzeichnet, dass**
die Anlenkschiene (20) einen Teleskopmechanismus hat und an der/den Abstützstreben (23) schwenkbar gehalten ist.

14. Halte- und Positioniervorrichtung nach einem der Ansprüche 11 bis 13, **dadurch gekennzeichnet, dass** die Anlenkschiene (20) zu einer Führungsschiene ausgebildet ist, an der Klemmelemente (26) längsverschiebbar und feststellbar angeordnet sind, die zur Anlenkung von Halte- und Positioniereinrichtungen für chirurgische Hilfsinstrumente und Optiken dienen.

## Claims

1. Holding and positioning device for surgical accessory instruments and optics with a mechanical link rod (1) at the free end portion of which a holder in the form of a ball (3) with a through bore (3a) for the accessory instrument or optic is arranged, [the said holder being] inserted in a bearing device fixed to the mechanical link rod (1) that exerts an adjustable gripping force on the ball (3), **characterized in that** the bearing device has a substantially U-shaped yoke (2) at the free arms of which axially aligned through bores for seating gripping means are formed, [the said yoke having] a bearing-shell-type ball-seat in which the ball (3) is gripped by the gripping means.

2. Holding and positioning device according to Claim 1, **characterized in that** the gripping means consist of two axially aligned holding rams (4, 4a) which grip the ball (3) between them.

3. Holding and positioning device according to Claim 2, **characterized in that** at least one of the rams (4) is axially displaceable by means of a handle (5) to vary the gripping force acting on the ball (3).

4. Holding and positioning device according to either of Claims 2 and 3, **characterized in that**
at least one of the rams (4) is preloaded in the axial direction by biasing means, in particular by one or more springs, by hydraulic, pneumatic, electrical or magnetic means, or by a combination of the aforesaid means.

5. Holding and positioning device according to any one of the preceding claims,
**characterized in that**
the centre of the ball (3) lies substantially on the central axis of the through bore (3a).

6. Holding and positioning device according to any one of the preceding claims,
**characterized in that**
the the holder is a sleeve, with the ball (3) fixed to its outer cylindrical surface.

7. Holding and positioning device according to any one of the preceding claims,
**characterized by**
a locking device for the surgical accessory instrument, consisting of a substantially U-shaped or bow-shaped yoke (12) with two parallel arms (14, 15), spaced apart, that offer axially aligned first and second through holes, and at least one clamping means (16) for individual, at least partial, adjustment of the diameter of one of the first and second through holes.

8. Holding and positioning device according to Claim 7, **characterized in that** the first through hole has a larger diameter than the second through hole.

9. Holding and positioning device according to either of Claims 7 and 8, **characterized in that**
the at least one clamping means (16) is a clamping screw reaching to the second through hole of smaller diameter.

10. Holding and positioning device according to any one of Claims 7 to 9,
**characterized in that**
the second through hole is radially enlargeable or openable in the manner of a clamp to afford lateral access to the through hole for insertion of a trocar.

11. Holding and positioning device according to any one of the preceding claims,
**characterized by**
a stand for anchoring a number of holding and positioning devices with
an anchor rail (20) which has at least at one end a support strut (23) provided with fixing means (24) for mounting the stand so that it straddles the operating table in the manner of a portal.

12. Holding and positioning device according to Claim 11, **characterized in that** the anchor rail (20) is vertically displaceable and lockable at the support strut(s) (23) arranged at one or both ends of the rail.

13. Holding and positioning device according to Claim 11 or Claim 12, **characterized in that**
the anchor rail (20) has a telescoping mechanism and is held pivotably on the support strut(s) (23).

14. Holding and positioning device according to any one of Claims 11 to 13,
**characterized in that**
the anchor rail (20) is configured as a guide rail on which clamp elements (26) are longitudinally displaceably and fixably arranged and serve to anchor holding and positioning devices for surgical accessory instruments and optics.

## Revendications

1. Un dispositif de maintien et de positionnement pour des instruments auxiliaires chirurgicaux et des optiques avec une tringlerie (1) mécanique, sur le tronçon d'extrémité libre duquel est disposé un support ayant la forme d'un sphère (3), présentant un perçage traversant (3a), pour l'instrument auxiliaire ou l'optique, le support étant inséré dans un dispositif de montage en palier, fixé sur la tringlerie (1) mécanique, qui exerce une force d'enserrement réglable sur la sphère (3), **caractérisé en ce que** le dispositif de montage en palier présente un cadre de maintien (2) sensiblement en forme de U, sur les branches libres duquel sont réalisés des perçages traversants en alignement axial, pour supporter des moyens d'enserrement, et présentant un siège de sphère du genre d'un coussinet de palier, dans lequel la sphère (3) est enserrée à l'aide des moyens d'enserrement.

2. Dispositif de maintien et de positionnement selon la revendication 1, **caractérisé en ce que** les moyens d'enserrement sont composés de deux tenons de maintien (4, 4a) en alignement axial, qui enserrent entre eux la sphère (3).

3. Dispositif de maintien et de positionnement selon la revendication 2, **caractérisé en ce qu'**au moins l'un des tenons (4) est déplaçable axialement à l'aide d'une manette (5), afin de faire varier la force d'enserrement sur la sphère (3).

4. Dispositif de maintien et de positionnement selon l'une des revendications 2 ou 3, **caractérisé en ce qu'**au moins l'un des tenons (4) est précontraint en direction axiale à l'aide d'un moyen de précontrainte, en particulier d'un ou plusieurs ressorts, d'un moyen hydraulique, pneumatique, électrique, magnétique, ou d'une combinaison des moyens cités ci-dessus.

5. Dispositif de maintien et de positionnement selon l'une des revendications précédentes, **caractérisé en ce que** le centre de la sphère (3) est situé sensiblement sur l'axe médian du perçage traversant (3a).

6. Dispositif de maintien et de positionnement selon l'une des revendications précédentes, **caractérisé en ce que** le support est une douille, sur l'enveloppe extérieure de laquelle est fixée la sphère (3).

7. Dispositif de maintien et de positionnement selon l'une des revendications précédentes, **caractérisé par** un dispositif de blocage pour l'instrument auxiliaire chirurgical, composé d'une fixation (12) sensiblement en forme de U ou en forme d'étrier, avec deux branches (14, 15) espacées parallèlement, constituant des premières et deuxièmes ouvertures traversantes en alignement axial, et au moins un moyen de serrage (16), pour le réglage individuel au moins partiel du diamètre d'une des premières et deuxièmes ouvertures de passage.

8. Dispositif de maintien et de positionnement selon la revendication 7, **caractérisé en ce que** la première ouverture de passage présente un plus grand diamètre que la deuxième ouverture de passage.

9. Dispositif de maintien et de positionnement selon l'une des revendications 7 et 8, **caractérisé en ce qu'**au moins un moyen de serrage (16) est une vis de serrage débouchant par un plus petit diamètre dans la deuxième ouverture de passage.

10. Dispositif de maintien et de positionnement selon l'une des revendications 7 à 9, **caractérisé en ce que** la deuxième ouverture de passage est susceptible d'être élargie radialement ou est susceptible d'être ouverte en forme de coquille, afin de créer un accès latéral dans l'ouverture de passage, pour effectuer une insertion d'un trocart.

11. Dispositif de maintien et de positionnement selon l'une des revendications précédentes, **caractérisé par** un pied pour articulation d'une pluralité de dispositifs de maintien et de positionnement, avec une glissière d'articulation (20), comprenant, sur au moins un tronçon d'extrémité, une entretoise d'appui (23) sur laquelle sont prévus des moyens de fixation (24) pour la fixation du pied, couvrant une table OP, à la façon d'un portique.

12. Dispositif de maintien et de positionnement selon la revendication 11, **caractérisé en ce que** la glissière d'articulation (20) est susceptible d'être réglée en hauteur sur une ou deux entretoises d'appui (23) disposées, côté extrémité, sur la glissière, et est susceptible d'être bloquée.

13. Dispositif de maintien et de positionnement selon la revendication 11 ou 12, **caractérisé en ce que** la glissière d'articulation (20) présente un mécanisme télescopique et est maintenue à pivotement sur la/les entretoise (s) d'appui (23).

14. Dispositif de maintien et de positionnement selon l'une des revendications 11 à 13, **caractérisé en ce que** la glissière d'articulation (20) est réalisée en une glissière de guidage, sur laquelle des éléments de serrage (26) sont disposés, d'une façon permettant un déplacement longitudinal et un blocage, les éléments de serrage servant à l'articulation des dispositifs de maintien et de positionnement pour des instruments auxiliaires chirurgicaux et des optiques.
